# EUROPEAN PATENT SPECIFICATION

(11) **EP 2 596 126 B1**
(45) Date of publication and mention of the grant of the patent: **30.11.2016**
(21) Application number: 11736082.6
(22) Date of filing: 21.07.2011
(51) Int. Cl.: C12Q 1/68

(54) **METHODS FOR DETERMINING A PROGNOSIS FOR SURVIVAL FOR A PATIENT WITH LEUKAEMIA**
VERFAHREN ZUR BESTIMMUNG EINER ÜBERLEBENSPROGNOSE VON PATIENTEN MIT LEUKÄMIE
MÉTHODES DE DÉTERMINATION D'UN PRONOSTIC DE SURVIE POUR UN PATIENT ATTEINT DE LEUCÉMIE

(30) Priority: 21.07.2010 NO 20101053
(43) Date of publication of application: 29.05.2013
(73) Proprietor: Bergen Teknologioverføring AS, 5006 Bergen (NO)
(72) Inventor: PENDINO, Frédéric, F-92220 Bagneux (FR); DE LA GRANGE, Pierre, F-94220 Charenton-le-Pont (FR); LILLEHAUG, Johan, R., N-5052 Bergen (NO); ALOYSIUS, Thomas, N-5178 Loddefjord (NO); KNAPPSKOG, Stian, N-5056 Bergen (NO)
(74) Representative: Kröncke, Rolf
(86) International application number: PCT/EP2011/062537
(87) International publication number: WO 2012/010661

(56) References cited:
- WO-A1-2010/085153
- WO-A2-2009/151337
- US-A1- 2007 154 931
- US-A1- 2009 118 132
- KALLIOPI N. MANOLA: "Cytogenetics of pediatric acute myeloid leukemia", EUROPEAN JOURNAL OF HAEMATOLOGY, vol. 83, no. 5, 1 November 2009 (2009-11-01), pages 391-405, XP55007845, ISSN: 0902-4441, DOI: 10.1111/j.1600-0609.2009.01308.x
- LEROY H ET AL: "CEBPA point mutations in hematological malignancies", LEUKEMIA, MACMILLAN PRESS LTD, US, vol. 19, no. 3, 1 March 2005 (2005-03-01), pages 329-334, XP002366039, ISSN: 0887-6924, DOI: 10.1038/SJ.LEU.2403614
- K. H. METZELER ET AL: "An 86-probe-set gene-expression signature predicts survival in cytogenetically normal acute myeloid leukemia", BLOOD, vol. 112, no. 10, 15 November 2008 (2008-11-15), pages 4193-4201, XP55007850, ISSN: 0006-4971, DOI: 10.1182/blood-2008-02-134411 cited in the application
- K H Metzeler ET AL: "Series GSE12417", NCBI, GEO , 22 August 2008 (2008-08-22), XP55007853, Retrieved from the Internet: URL:http://www.ncbi.nlm.nih.gov/geo/query/ acc.cgi?acc=GSE12417 [retrieved on 2011-09-22]
- J. P. RADICH: "Gene expression changes associated with progression and response in chronic myeloid leukemia", PROCEEDINGS OF THE NATIONAL ACADEMY OF SCIENCES, vol. 103, no. 8, 1 January 2006 (2006-01-01), pages 2794-2799, XP55007856, ISSN: 0027-8424, DOI: 10.1073/pnas.0510423103 cited in the application
- GAIDZIK VERENA I ET AL: "RUNX1 mutations in acute myeloid leukemia: results from a comprehensive genetic and clinical analysis from the AML study group", JOURNAL OF CLINICAL ONCOLOGY,, vol. 29, no. 10, 1 April 2011 (2011-04-01) , pages 1364-1372, XP009152359, ISSN: 1527-7755 cited in the application
- L Bullinger: "Series GSE23312", NCBI, GEO , 31 December 2010 (2010-12-31), XP55007863, Retrieved from the Internet: URL:http://www.ncbi.nlm.nih.gov/geo/query/ acc.cgi?acc=GSE23312 [retrieved on 2011-09-22]
- ULRIKE BACHER ET AL: "Current status of gene expression profiling in the diagnosis and management of acute leukaemia", BRITISH JOURNAL OF HAEMATOLOGY, vol. 145, no. 5, 1 June 2009 (2009-06-01), pages 555-568, XP55007871, ISSN: 0007-1048, DOI: 10.1111/j.1365-2141.2009.07656.x
- BULLINGER L ET AL: "USE OF GENE-EXPRESSION PROFILING TO IDENTIFY PROGNOSTIC SUBCLASSES IN ADULT ACUTE MYELOID LEUKEMIA", NEW ENGLAND JOURNAL OF MEDICINE, MASSACHUSETTS MEDICAL SOCIETY, BOSTON, MA, US, vol. 350, no. 16, 15 April 2004 (2004-04-15), pages 1605-1616, XP009059431, ISSN: 1533-4406, DOI: 10.1056/NEJMOA031046
- HUANG TIEN-SHENG ET AL: "LEDGF/p75 has increased expression in blasts from chemotherapy-resistant human acute myelogenic leukemia patients and protects leukemia cells from apoptosis in vitro", MOLECULAR CANCER, BIOMED CENTRAL, LONDON, GB, vol. 6, no. 1, 23 April 2007 (2007-04-23), page 31, XP021024416, ISSN: 1476-4598, DOI: 10.1186/1476-4598-6-31
- Anonymous: "HGNC report for CXXC5", Hugo gene nomenclature committee , 2011, XP55007763, Retrieved from the Internet: URL:http://www.genenames.org/data/hgnc_dat a.php?hgnc_id=26943 [retrieved on 2011-09-22]
- ASTORI ANDREY ET AL: "CXXC5 (Retinoid-Inducible Nuclear Factor, RINF) is a Potential Therapeutic Target in High-Risk Human Acute Myeloid Leukemia", ONCOTARGET, vol. 4, no. 9, September 2013 (2013-09), pages 1438-1448,

## Description

### FIELD OF THE INVENTION

The present disclosure relates to a method for determining a prognosis for survival or the progression of the disease for a patient with leukaemia, the risk of resistance to treatment, or the risk of a relapse of leukaemia, like an acute myeloid leukaemia, e.g. an acute myeloid leukaemia with normal karyotype or a CBF (Core-Binding-Factor) acute myeloid leukaemia, and a method for monitoring the effectiveness of a course of treatment for a patient with leukaemia, as well as a kit for use in said methods, preferably, by quantifying RINF mRNA expression.

### BACKGROUND FOR THE INVENTION

Identification of genes specifically deregulated in tumour cells could bring to light new tumour suppressors and oncogenes with potential clinical applications in diagnostics, prognostics, and therapeutics. In PCT application WO 2009/151337 a new target gene of retinoids, *CXXC5,* encoding a nuclear factor has been described that was functionally characterized for the first time at the mRNA and protein level and named RINF (Retinoid-Inducible Nuclear Factor). In PCT application WO 2010/085153 it is described for the first time that measurement of RINF mRNA in breast cancer tumours, could be informative for overall survival of breast cancer patients.

Present data indicate that RINF plays an essential role during *in vitro* human hematopoiesis. Indeed, expression studies and gene silencing experiments both demonstrate RINF implication during terminal differentiation of myeloid leukaemia cells (NB4 and HL60 cell lines), but also during myelopoiesis of normal hematopoietic progenitors (bone marrow CD34+ cells). In keeping with its essential role during terminal myeloid differentiation and its localization to chromosome 5q31.3, a region often deleted in myeloid leukaemia (more especially in acute myeloid leukaemia and myelodysplasia), RINF has been suggested as a promising tumour suppressor candidate at that locus in some myeloid malignancies.

Because RINF expression is not restricted to the hematopoietic tissue and may also be involved in development and/or homeostasis of other tissues, RINF expression in some solid tumours derived from different tissues have been investigated. It is shown in PCT application WO 2009/151337 that this gene could have a deregulated expression in some malignant tissues compared to their normal tissues of origin. Indeed, RINF mRNA expression was examined in solid tumour samples from patients suffering from breast cancer, and RINF expression was significantly higher in breast tumours compared to normal breast tissues controls.

Further, in PCT application WO 2010/085153 it is shown that measurement of RINF mRNA in breast cancer tumours, could be informative for overall survival of these breast cancer patients. In this application, it is also described a "kit" for detection of RINF mRNA by quantitative RT-PCR.

Leukaemia (American English) or leukaemia (Standard English; Greek leukos *λευκóς,* "white"; aima *αíμα,* "blood") is a cancer of the blood or bone marrow characterized by an abnormal increase of blood cells, usually leukocytes (white blood cells).

The major forms of leukaemia can be divided into four main categories. Myelogenous and lymphocytic/lymphoblastic leukaemias, each have acute and chronic forms. The terms myelogenous or lymphocytic/lymphoblastic denote the cell type involved. In addition, within each category, distinct leukaemias are defined according to a combination of morphology, immunophenotype, and cytogenetic features in addition to clinical symptoms.

Acute leukaemia is a clonal disease of hematopoetic stem cells. For example, in the United States more than half of all new leukaemias are acute leukaemias. Said acute leukaemias can be differentiated into acute myelogenous leukaemia (AML), also called acute non-lymphocytic leukaemia and acute lymphocytic or acute lymphoblastic leukaemia (ALL) based on whether hematopetic stem cells have been differentiated along the lymphoid or myeloid lineage. In addition, chronic types of leukaemia are differentiated into chronic myelogenous leukaemia (CML) or chronic lymphocytic/lymphoblastic leukaemia (CLL).

Among leukaemia, acute myeloid leukaemia (AML) represents a distinct group of disease with heterogeneous genetic basis, pathophysiology, and prognosis. In the past, the karyotype of the AML cells has emerged as the most important prognostic factor, and patients can be classified into favourable, intermediate, and unfavourable prognostic groups according to the number and the specific types of chromosomal aberrations. However, almost half of all adult AML patients present with a normal karyotype at diagnosis. These patients are usually assigned to the intermediate prognostic group. More recently, mutations in specific genes that allow the identification of prognostic subgroups in cytogenetically normal (CN) AML have been described. Internal tandem duplications (ITD) of the fms-like tyrosine kinase 3 *(FLT3)* gene are strong predictors of inferior outcome. Mutations in the nucleophosmin 1 (*NPM1*) gene lead to cytoplasmic localization of the NPM protein and are a favourable prognostic factor, especially in patients lacking *FLT3* ITD. Other prognostically relevant alterations include mutations in the *MLL* and *CEBPA* genes as well as overexpression of *WT1, MN1, BAALC, ERG,* and *EVI1(MECOM).* However, in approximately 24% of CN-AML cases, none of the aforementioned mutations can be detected. These findings illustrate that CN-AML itself constitutes a heterogeneous disease and that there probably are many genetic alterations in CN-AML to be discovered that affect response to treatment and survival.

In the case of leukaemia, like ALL, AML, CLL and CML, and especially in the case of AML and CN-AML, but also with CBF-AML, these prognostic indicators are very important to help the physicians to distinguish the group of patients that would more likely benefit from a chemotherapy (usually, disease with a favourable prognosis) from patients that (more probably) would not, and for instance, that should be considered for autologous or allogenic stem cell transplantation (these patients present usually with unfavourable prognosis and age below 60). In other terms, prognostic factors are of very high potential for the treatment decision making in these malignant haemopathies and additional prognostic markers are needed.

### Summary of the present invention

The inventors have now surprisingly found that the level of RINF expression correlates with the survival of patients with leukaemia, the progression of the disease, the risk of relapse of leukaemia more precisely an acute myeloid leukaemia, more precisely an acute myeloid leukaemia with normal karyotype.

The present invention thus relates to methods for determining the prognosis for survival, the progression of the disease, or the risk of relapse of acute myeloid leukaemia, as defined in the claims. The disclosure also relates to a kit for determining the prognosis for survival, and for testing the effect of treatment regimens.

### Brief description of the drawings

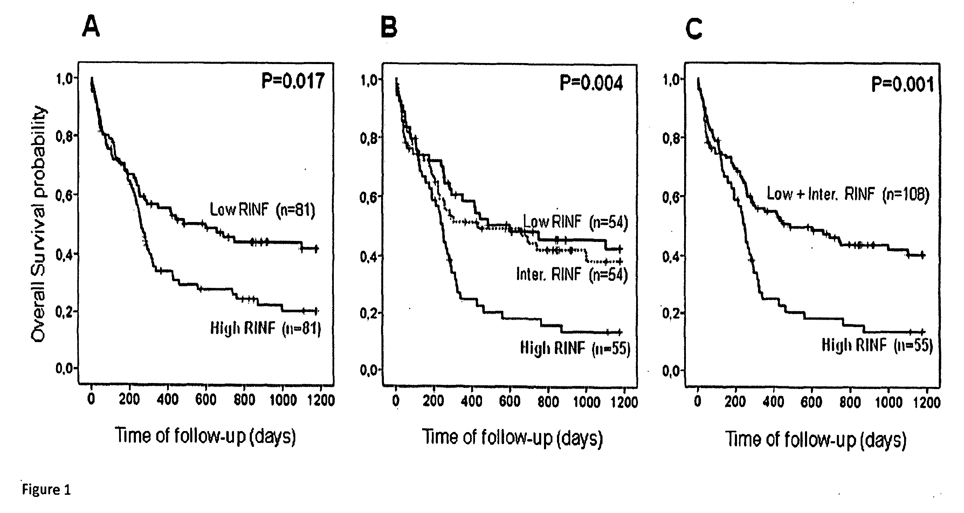

Figure 1: **High *RINF* mRNA expression at diagnosis is associated with a shorter overall survival for AML patients with normal karyotype CN-AML (n=163).**
   The microarray dataset (Affymetrix GeneChip Human Genome HG-U133B) performed by Metzeler KH, et al., Blood. 2008 Nov 15;112(10):4193-201 (n=163 patient samples) was downloaded from the Gene Expression Omnibus website (http://www.ncbi.nlm.nih.gov/geo/) with accession number GSE12417. The whole raw data were normalized using RMA (Robust Multiarray Averaging method) with the Expression Console software from Affymetrix. The *CXXC5* mRNA expression was then calculated as the mean intensity of the 3 probesets targeting *CXXC5* (222996_s_at, 224516_s_at and 233955_x_at). The patients have been classified in two equivalent groups **(A),** according to an expression above (High, n=81) or below (Low, n=81) the median, or in 3 equivalent groups **(B and C),** according to a High (n=55), an Intermediary (n=54), or a Low (n=54) *RINF* expression level. For panel C, the Low (n=54) and Intermediary (n=54) groups have been fused. Note that because of an odd number of patients (n=163), the patient at the median has been censored for the 2 groups stratification and were not exactly equivalents for the 3 groups stratification (n=55 *versus* 54). The patient groups with high or low *CXXC5* mRNA (RINF) were then correlated with the clinical outcomes concerning survival that were also accessed through the Gene Expression Omnibus website. The Kaplan-Meier curves (for survival analysis) and the log-rank test were performed by using the statistical SPSS 15.0. *P* values (log-rank test) of the comparison of the various groups of patients are indicated in the panels.

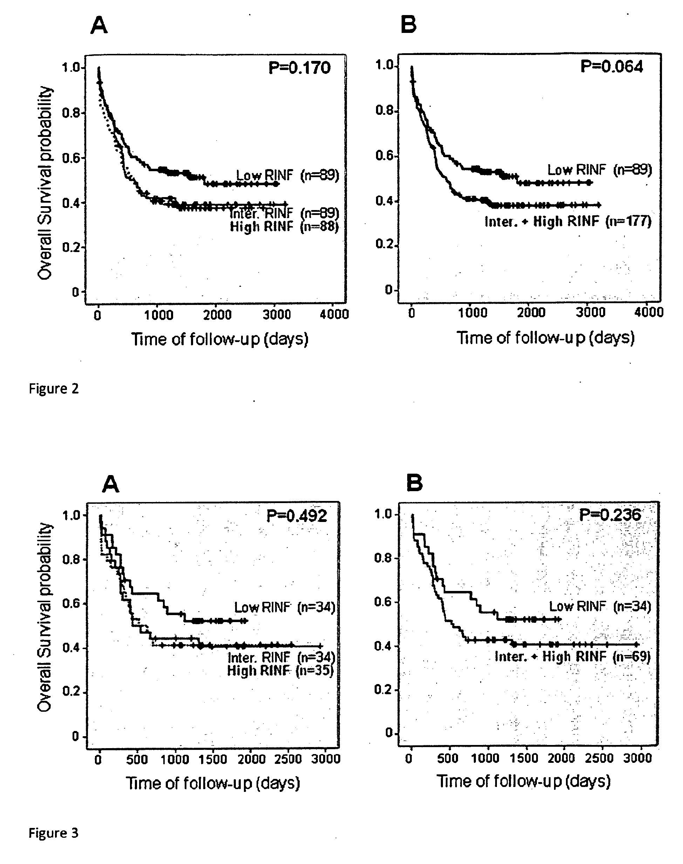

Figure 2: **Low *RINF* mRNA expression is related to a better prognosis in AML, all cytogenetic subgroups considered altogether (n=266).**
   The microarray datasets (Standford microarrays) performed by Gaidzik VI, et al., J Clin Oncol. 2011 Apr 1;29(10):1364-72 (n=269, but n=266 patient samples with *CXXC5* expression data available) was downloaded from the Gene Expression Omnibus website (http://www.ncbi.nlm.nih.gov/geo/) with accession number GSE23312. Fluorescence ratios were normalized by mean-centering genes for each array and then mean-centering each gene across arrays. The *CXXC5* mRNA expression was then extracted and patients have been classified in three equivalent groups **(A and B),** according to a High (n=88), an Intermediary (n=89), or a Low (n=89) *RINF* expression level. For panel B, the High (n=88) and Intermediary (n=89) groups have been fused (n=177). Note that the patient number were not exactly equivalents for the 3 groups stratification (n=89, 89 *versus* 88) since the total number of patients was not a multiple of 3 (n=266). The patient groups with high, Intermediary, and low *CXXC5* mRNA were then correlated with the clinical outcomes concerning survival that were also accessed through the Gene Expression Omnibus website. The Kaplan-Meier curves (for survival analysis) and the log-rank test were performed by using the statistical SPSS 15.0. P values (log-rank test) of the comparison of the various groups of patients are indicated in the panels.
Figure 3: **Low *RINF* mRNA expression is related to a better prognosis in AML with normal karyotype (n=102). The** microarray datasets (Standford microarrays) performed by Gaidzik VI *et al.* (see above) was used and analysed as described above. The *CXXC5* mRNA expression was then extracted and patients have been classified in three equivalent groups **(A and B),** according to a High (n=35), an Intermediary (n=34), or a Low (n=34) *RINF* expression level. For panel B, the High (n=35) and Intermediary (n=34) groups have been fused (n=69). Note that the patient number were not exactly equivalents for the 3 groups stratification (n=34, 34 *versus* 35) since the total number of patients was not a multiple of 3 (n=102). The patient groups with High, Intermediary, and Low *CXXC5* mRNA were then correlated with the clinical outcomes concerning survival that were also accessed through the Gene Expression Omnibus website. The Kaplan-Meier curves (for survival analysis) and the log-rank test were performed by using the statistical SPSS 15.0. P values (log-rank test) of the comparison of the various groups of patients are indicated in the panels.

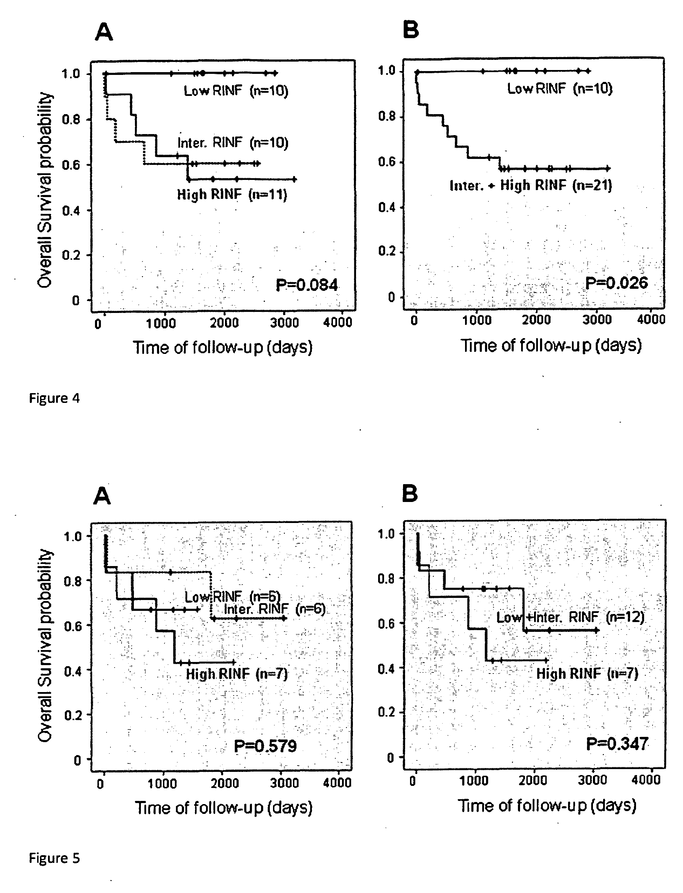

Figure 4: **Low *RINF* mRNA expression is related to a better prognosis in AML with inversion (16) (n=31).** The microarray datasets (Standford microarrays) performed by Gaidzik VI *et al.* was obtained and analysed as described above. The *CXXC5* mRNA expression was then extracted and patients have been classified in three equivalent groups **(A and B),** according to a High (n=11), an Intermediary (n=10), or a Low (n=10) *RINF* expression level. For panel B, the High (n=11) and Intermediary (n=10) groups have been fused (n=21). Note that the patient number were not exactly equivalents for the 3 groups stratification (n=10, 10 *versus* 11) since the total number of patients was not a multiple of 3 (n=31). The patient groups with high, Intermediary, and low *CXXC5* mRNA were then correlated with the clinical outcomes concerning survival that were also accessed through the Gene Expression Omnibus website. The Kaplan-Meier curves (for survival analysis) and the log-rank test were performed by using the statistical SPSS 15.0. P values (log-rank test) of the comparison of the various groups of patients are indicated in the panels
Figure 5: **Low *RINF* mRNA expression is related to a better prognosis in AML with translocation t(8;21) (n=19).** The microarray datasets (Standford microarrays) performed by Gaidzik VI *et al.* (n=269, but n=19 patient samples with *CXXC5* expression data available and translocation t(8;21)) was analysed as described above. The *CXXC5* mRNA expression was then extracted and patients have been classified in three equivalent groups **(A and B),** according to a High (n=7), an Intermediary (n=6), or a Low (n=6) *RINF* expression level. For panel B, the Low (n=6) and Intermediary (n=6) groups have been fused (n=12). Note that the patient number were not exactly equivalents for the 3 groups stratification (n=6, 6 *versus* 7) since the total number of patients was not a multiple of 3 (n=19). The patient groups with high, Intermediary, and low *CXXC5* mRNA were then correlated with the clinical outcomes concerning survival that were also accessed through the Gene Expression Omnibus website. The Kaplan-Meier curves (for survival analysis) and the log-rank test were performed by using the statistical SPSS 15.0. P values (log-rank test) of the comparison of the various groups of patients are indicated in the panels

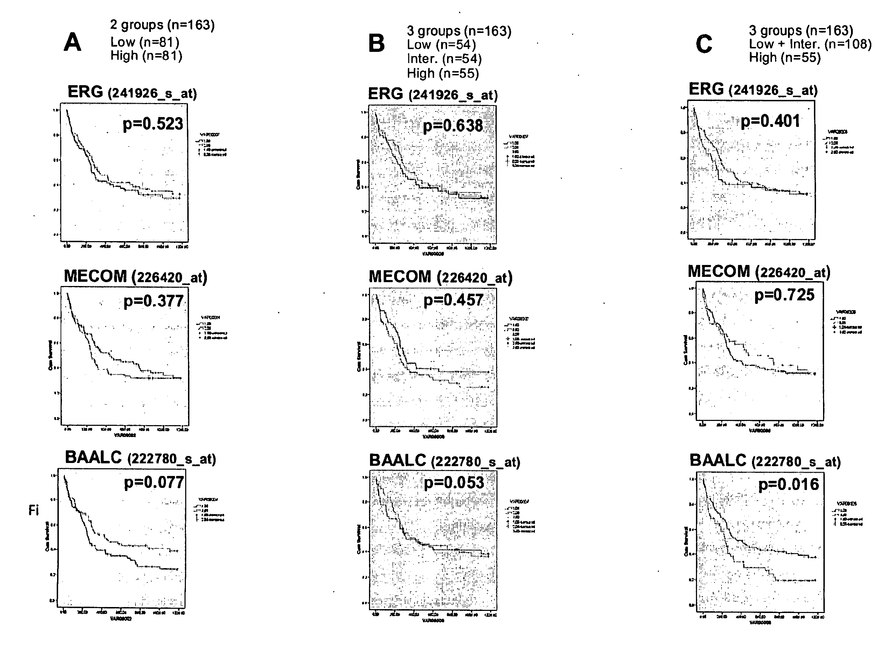

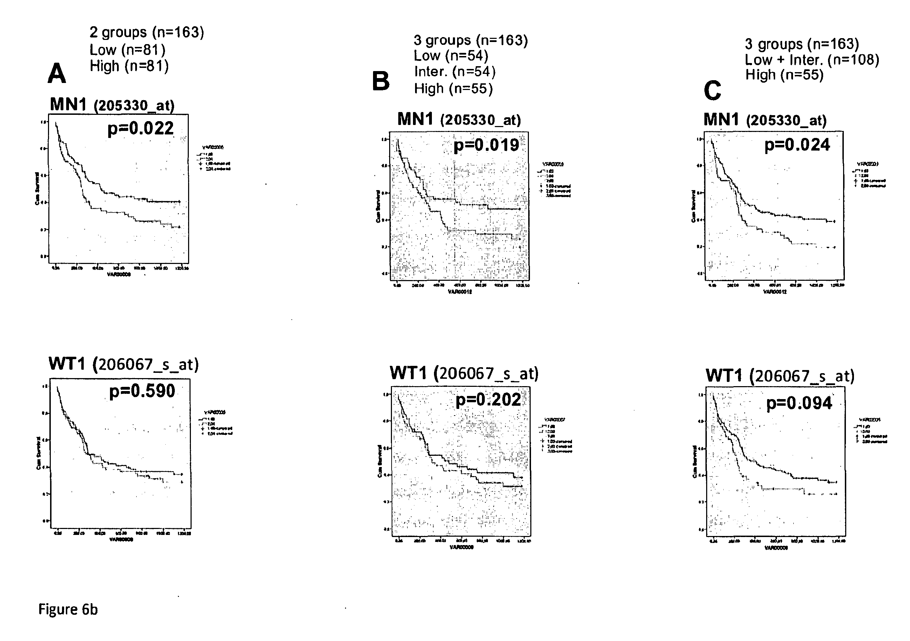

Figure 6: Figure 6 relates to the survival rate for marker molecules described in the art as suitable marker molecules in leukaemia. The cohort was classified in two or three groups, respectively, based on the expression levels of the respective marker. (A) ERG, MECOM BAALC, (B) MN1, WT1.

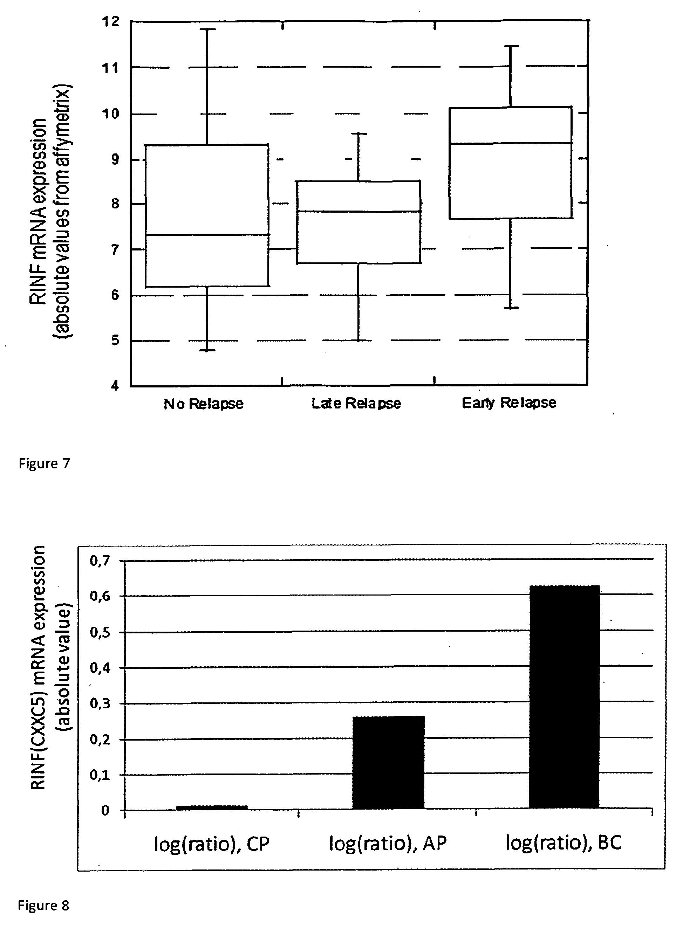

Figure 7: In Figure 3 RINF mRNA expression (at a diagnosis time) is higher in the group of acute lymphocytic leukaemia patients (n=197) that relapse after treatment than in persons showing no relapse.
Figure 8. RINF mRNA expression is increased during the progression of Chronic Myeloid Leukemia from chronic phase (CP) to blast crisis (BC). The RINF (CXXC5) gene expression data were extracted from a gene list (supporting table 4 or 10423Table4.xls) available online at the PNAS website (http://www.pnas.org/content/103/8/2794/suppl/DC1). CP: chronic phase (n=42), AP: accelerated phase by blast count criteria (n=9) or by the occurrence of additional clonal cytogenetic changes (n=8), BC: blast crisis (n=28).

### Detailed description of the present invention

In a first aspect, the present invention relates to a method for determining the progression of the disease or a prognosis for survival of a patient with acute myeloid leukaemia, or the risk of a relapse of acute myeloid leukaemia in a patient diagnosed and, optionally, treated for acute myeloid leukaemia, characterized in that said method comprising:
a) determining the level of RINF mRNA expression in a biological sample from said patient,
b) comparing the level of RINF mRNA expression in said sample to a reference level of RINF,
whereby a level of RINF mRNA expression in said sample of said patient lower than the reference level correlates with better prognosis and increased survival of said patient, slower progression of the disease or lower risk of a relapse, and whereby a level of RINF mRNA expression in said sample higher than the reference level correlates with decreased survival and bad prognosis, faster progression of the disease or higher risk of relapse.

As used herein, the term "subject" or "individual" or "patient" which is used herein interchangeably refers to an individual or a subject or a patient in need of a therapy or suspected to be afflicted with a condition or disease, namely leukaemia. Preferably, the subject or individual is a vertebrate even more preferred a mammal, particularly preferred a human.

The method according to the present disclosure is preferably conducted by collecting a sample from the individual and determining *in vitro* the level of RINF using appropriate means.

The term "control sample" or "reference sample" as used herein refers e.g. to a sample of an individual of the same species which individual is not suffering from leukaemia. Alternatively, the "control sample" or "reference sample" is a sample representing the median of a panel of patients afflicted with leukaemia. In another embodiment, the "control sample" is a cell line sample (for instance such as NB4, HL60, K562, MCF7).

A sample according to the invention is a biological material, which has been obtained from an individual. The term "sample" and "biological sample" are used herein interchangeably. In particular, said terms include material obtained from blood, plasma, tissue, bone marrow, serum or tumour, tumour biopsy or neoplastic cell containing sample.

The expression "level" or "amount" is meant to describe the relative level of said molecules according to the present invention relative to the same molecule in a control sample or reference sample, or the absolute amount of said molecules according to the present invention. Relative means that no distinct amounts such as mole or milligram per litre etc. are stated, but that for example is stated the sample contains more, less or the same amount of a certain molecule as compared to a control sample. The term "more, less or the same amount" in this situation includes also arbitrary units.

With "elevated" or "higher" and on the other side "decreased" or "lower" according to the invention is meant, that the molecule is present in a sample in higher (or lower, respectively) quantities, or amounts or concentration, as compared to another sample, or as compared to a control sample or as compared to a reference sample or as compared to a reference value, regardless if these measurements are relative or absolute measurements. Preferably, the molecule which is increased or decreased is present in a concentration, or quantity or amount which is at least 10 %, like at least 20 %, e.g. at least 30 %, or 50% above or below the value to which it is compared. In other words, preferably, the molecule is increased or decreased at least 2 fold, e.g. 3 fold, 4 fold or even 5 fold or more above or below the level and/or amount in the control or reference sample. For example, the molecule in the sample is higher than 4.5 fold time the level of NB4 cells measured in the reference sample.

With "stratification of a subject" according to the invention is meant to determine the therapy regimen for the treatment of leukaemia. In particular, said stratification includes determining whether said subject will benefit from a specific treatment, irradiation chemotherapy, or stem cell transplantation.

Prognosis is a medical term to describe the likely outcome of an illness. Here, a good "prognosis for survival" correlates with high rate survival of the patients and "bad prognosis for survival" correlates with a low rate of survival of the patient group.

Thus, in one aspect, the present invention relates to a method for determining a prognosis for survival for a patient with acute myeloid leukaemia, wherein said method comprising:
(a) determining a measure for the level of RINF mRNA expression in a biological sample from said patient,
(b) comparing the level of RINF mRNA expression in said sample to a reference level of RINF,
wherein a level of RINF expression lower than the reference level in said sample correlates with increased survival probability or rate of said patient, and wherein a level of RINF expression higher than the reference level in said sample correlates with decreased survival probability or rate.

In this connection, the terms "a measure for the level" and "level" are used interchangeably herein.

In a preferred embodiment, said biological sample is whole blood, serum, plasma, liquor, bone marrow, a tumour, or a tumour biopsy or neoplastic cell containing sample. In fact, the biological sample can be a fraction of blood or bone marrow. for instance, it is a preferably PBMC (peripheral blood mononuclear cells) or BMMC (bone marrow mononuclear cells), isolated after ficoll-density gradient separation, and/or CD34+ cell sorting enrichment.

A preferred embodiment relates to acute myeloid leukaemia (AML). Moreover, it is preferred that the leukaemia is a leukaemia with normal karyotype or CBF, more preferable an acute myeloid leukaemia with normal karyotype.

In a preferred embodiment said measure for the level of RINF expression is determined by quantification of the expression of mRNA which encodes for a RINF protein, or a quantification of a nucleic acid comprising the sequence of SEQ ID NO1 or SEQ ID NO 2, or a functional fragment or variant thereof, or a functionally equivalent isolated DNA sequence hybridizable thereto.

In this connection, the term "hybridizable thereto" refers to conditions wherein sequences hybridize under stringent hybridization conditions (see, T. Maniatis et al., Cold Spring Harbor Laboratory (1982), molecular Cloning/ a laboratory manual, pages 387 to 389) to the sequences of Seq ID. No. 1 or 2. An example of stringent hybridization condition is hybridization at 4xSSC at 65°C, followed by a washing in 0.1xSSC at 65°C for an hour. Alternatively, an exemplary stringent hybridization condition is in 50% formamide, 4xSSC at 42 °C.

Further, the term "functional fragment" refers to fragments of Seq. ID. Nos. 1 or 2 having the same functionality as the polypeptide encoded by Seq. ID. Nos. 1 or 2, respectively. Moreover, the term "variant" refers to molecules having the same functionality as the nucleic acid molecules of Seq. ID Nos. 1 and 2 or the polypeptides encoded by said sequences, like the polypeptide of Seq. ID. No.3.

In a further preferred embodiment is the level of RINF expression relative to said reference level used to determine a proper course of treatment for said patient.

In a preferred embodiment is the level of RINF expression determined by quantitative RT-PCR. More preferable, said quantitative RT-PCR uses oligonucleotides targeting SEQ ID NO 1 or SEQ ID NO 2 for determining the RINF expression, and more preferable said RT-PCR uses primers amplifying exon 3 and 4, or exon 1 and 2 of SEQ ID NO 3.

Preferable, the level of RINF expression is determined based on the relative expression of RINF normalized to the expression of the rpP2 gene, also known as RPLP2 ribosomal protein, large, P2, (HGNC: 10377).
Preferable, the primers for the detection of SEQ ID NO 1 or 2 are
5'-tccgctgctctggagaag-3' (Seq. ID No. 4), 5'-cacacgagcagtgacattgc-3' (Seq. ID No. 5) and 6-FAM-aacccaaagctgccctctcc-BBQ (Seq. ID No. 8).

Preferable, the primers for the detection of rpP2 are 5'-atgcgctacgtcgcc-3' (Seq. ID No. 6), 5'-ttaatcaaaaaggccaaatcccat-3' (Seq. ID No. 7) and Cy5-agctgaatggaaaaaacattgaagacgtc-BBQ (Seq. ID No. 9).

6-FAM is a reactive water-soluble fluorescent dye. It is an isomer of carboxyfluorescein, widely used to label oligonucleotides for real-time PCR applications. 6-FAM is excited maximally at 492 nm and emits maximally at 517 nm.

Cy5 (Cyanine-5) is a reactive water-soluble fluorescent dye of the cyanine dye family. Cy5 is excited maximally at 649 nm and emits maximally at 670 nm, in the far red part of the spectrum.

BBQ means BlackBerry Quencher is a "dark quencher" for every fluorescent dye. Non-fluorescent quenchers are fundamental for multiplex applications with more than one dye. A dark quencher is a substance that absorbs excitation energy from a fluorophore and dissipates the energy as heat (while a typical (fluorescent) quencher re-emits much of this energy as light).

Of course, any other marker or label may be used for effecting the analysis accordingly. The skilled person is well aware of suitable label or marker molecules.

Preferable, the PCR amplification is conducted by an initial denaturation at 95°C for 5 min, and then the samples run through 44 cycles of the following conditions: denaturation for 10 seconds at 95 °C and elongation at 55 °C for 20 seconds.

In a further aspect the level of RINF expression is determined immunochemically based on an antibody - based detection system. Said antibody can be a polyclonal antibody, and is more preferable a monoclonal antibody. Preferable, said antibody is interacting with an epitope of a protein or protein sequence of SEQ ID NO 3, and more preferable said antibody is interacting with the amino acids 45-48 of SEQ ID NO 3.

In a further aspect, the RINF expression is measured by a DNA, RNA or protein array.

Suitable methods to detect the level, e.g. the relative level and/or absolute amount of RINF in the samples are well known in the art. For example, suitable methods include suitable mass spectrometry methods, e.g. matrix assisted laser desorption ionisation (MALDI), continuous or pulsed electrospray ionisation (ESI) and related methods such as ionspray or thermospray or massive cluster impact. The ion sources can be matched with detection formats including linear or non-linear reflection time of light (TOF), single or multiple quadrupole, single or multiple magnetic sector, Fourier transformation ion cyclotron resonance (FTICR), ion trap, and combinations thereof. Other mass spectrometic methods suitable are for example fast atom bombardment (FAB), mass spectrometry, surface enhanced laser desorption (ionisation) (SELDI), masssspectrometry, etc.

Further, suitable immunologic methods among other are enzyme link immunoassays (ELISA), sandwich, direct, indirect, or competitive ELISA assays, enzyme link immunospotassays (ELIspot), radio immunoassays (RIA), flow-cytometry assays (FACS), immunohistochemistry, Western Blot, fluorescence resonance energy transfer (FRET) assays, protein chip assays using for example antibodies, antibody fragments, receptor ligands or other agents binding the molecules of the disclosure.

Among other Northern Blot or Southern Blot hybridization, nucleic acid dot or slot Blot Hybridization, in situ hybridization, nucleic acid chip assays (for example using RNA, DNA or other nucleic acids to specifically capture the nucleic acid of interest), polymerase chain reaction (PCR), reverse transcriptase-PCR (RT-PCR), real time PCR, and in particular, real time quantitative polymerase chain reaction (RQ-PCR) after reverse transcription may be used as molecular biology methods to detect the molecules of the invention.

Further methods, such as nuclear magnetic resonance (NMR), fluorometry, colorimetry, radiometry, luminometry or other spectrometric methods, liquid chromatography, capillary chromatography, thin-layer chromatography, plasmon resonance, one-or-two gel dimensional electroporesis etc. can be used to detect the molecules of the invention.

In another preferred embodiment the level and/or amount of RINF is determined on mRNA level. Particular preferred the determination is conducted by RQ-PCR, for example applying known real time PCR-methods. Another preferred aspect relates to the determination of the level and/or amount of RINF on protein level. For example, said level may be determined by immunological methods as outlined above using specific anti-RINF antibodies.

In a further embodiment, said method further comprises a step (c); (c) classifying said patient as belonging to either a first or a second group of patients, wherein said first group of patients having levels of RINF expression lower than the reference level is classified as having an increased likelihood of survival, slower progression of the disease, better prognosis or lower risk or relapse compared to said second group of patients having levels of RINF expression higher than the reference level.

In a further embodiment, said method further comprises a step (c);
(c) classifying said patient as belonging to either a first or a second group of patients, wherein said first group of patients having levels of RINF expression lower than said second group of patients, and where the first group of patients is classified as having an increased likelihood of survival, slower progression of the disease, better prognosis or lower risk or relapse compared to said second group of patients.

A second aspect of the present invention relates to a method for monitoring the effectiveness of a course of treatment for a patient with acute myeloid leukaemia, wherein said method comprising:
(a) determining a level of RINF mRNA expression in a biological sample from said patient prior to treatment, and
(b) determining the level of RINF mRNA expression in a neoplastic cell-containing sample from said patient after treatment, whereby comparison of the level of RINF expression prior to treatment with the level of RINF expression after treatment indicates the effectiveness of said treatment, wherein a decrease in the level of RINF expression after treatment indicates that treatment is effective against said leukaemia.

In addition, the present disclosure relates to a method for the stratification of the patient being afflicted with leukaemia to determine the therapy regimen for the treatment of leukaemia comprising
a) the relative level and/or the absolute amount of RINF in a sample from said patient;
b) comparing the level and/or amount of RINF to the level and/or amount of RNIF in a control sample, with the level and/or amount of RINF in samples obtained prior to the begin of the therapy of said patient, or obtained in earlier stages of the therapy regimen of said patient.

A third aspect of the present disclosure relates to a use in accordance with any of the method claims, wherein at least one detection member for RINF is used in a kit for determining a prognosis for survival for a patient with leukaemia.

A forth aspect of the present disclosure relates to a kit for determining a prognosis for survival for a patient with leukaemia, characterized in that said kit comprises compounds capable of detecting the level of RINF expression in a biological sample.

Preferable aspects for the second, third and forth aspects (independently for each aspect) are indicated below:
- said leukaemia is AML, ALL, CML, or CLL, preferably acute myeloid leukaemia, and more preferable acute myeloid leukaemia with normal karyotype or CBF (Core-binding-factor) (CBF-AML are characterized by translocation t(8:21) or inversion inv(16), respectively). CBF-AML respond usually well to chemotherapy. However, some of these patients do not respond well to therapy and it has been recognized that this groups is characterized by high RINF expression.
- said biological sample is is obtained from bone marrow, blood or tissue, like plasma, serum, liquor, a tumour, or a tumour biopsy or neoplastic cell containing sample.
- said measure for the level of RINF expression is determined by quantification the expression of mRNA which encodes for a RINF protein, or a quantification of a nucleic acid comprising the sequence of SEQ ID NO1 or SEQ ID NO 2, or a functional fragment or variant thereof, or a functionally equivalent isolated DNA sequence hybridizable thereto.
- the level of RINF expression relative to said reference level is used to determine a proper course of treatment for said patient.
- the level of RINF expression is determined by quantitative RT-PCR. More preferable, said quantitative RT-PCR uses oligonucleotides also termed Hydrolysis Probes targeting SEQ ID NO 1 or SEQ ID NO 2 for determining the RINF expression, and more preferable said RT-PCR uses primers amplifying exon 3 and 4, or exon 1 and 2 of SEQ ID NO 3.

In this connection, the term "Hydrolysis probes" are used to define sequence specific DNA oligonucleotides that are chemically modified (labeled with both a fluorescent dye and a quencher dye) in order to easily monitor the concentration of a specific DNA during a quantitative real-time Polymerase Chain Reaction (qRT PCR). The fluorescence emitted (when excited by an external light source at each PCR cycle) is proportional to the amount of the specific DNA product formed.

The Hydrolysis Probe chemistry relies on the 5'-3' exonuclease activity of Taq polymerase, which degrades a hybridized non-extendible DNA probe during the extension step of the Polymerase Chain Reaction (PCR). This probe is designed to specifically hybridize to a region within the amplicon and is dual-labeled with a fluorecent dye and a quencher. The close proximity of the quencher suppresses the fluorescence of the reporter dye. Once the exonuclease activity of Taq polymerase degrades the probe, the fluorescence of the reporter increases at a rate that is proportional to the amount of template present.
- the level of RINF expression is determined based on the relative expression of RINF normalized to the expression of the rpP2 gene.
- the primers for the detection of SEQ ID NO 1 or 2 are
   5'-tccgctgctctggagaag-3' (Seq. ID No. 4), 5'-cacacgagcagtgacattgc-3' (Seq. ID No. 5) and 6-FAM-aacccaaagctgccctctcc-BBQ (Seq. ID no. 8).
- the primers for the detection of rpP2 are 5'-atgcgctacgtcgcc-3' (Seq. ID No. 6), 5'-ttaatcaaaaaggccaaatcccat-3' (Seq. ID No. 7) and Cy5-agctgaatggaaaaaacattgaagacgtc-BBQ (Seq. ID No. 9).
- the PCR amplification is conducted by an initial denaturation at 95°C for 5 min, and than the samples is run through 44 cycles of the following conditions: denaturation for 10 seconds at 95 °C and elongation at 55 °C for 20 seconds.
- the level of RINF expression is determined immunochemically based on an antibody. Said antibody can be a polyclonal antibody, and is more preferable a monoclonal antibody. Preferable, said antibody is interacting with an epitope of a protein or protein sequence of SEQ ID NO 3, and more preferable said antibody is interacting with the amino acids 45-48 of SEQ ID NO 3.
- the RINF expression is measured by a DNA, RNA or protein array.

### EXPERIMENTAL SECTION

### Materials and methods

### Quantitative RT-PCR of RINF

First-strand cDNA synthesis (RT) was carried out starting with total RNA (1 µg) in a 20 µl volume using oligo-dT primers with Transcriptor Reverse Transcriptase (Roche, Cat. N° 05 531 287 001) in accordance with the manufacturer's instructions. Quantitative PCRs were performed using specific Hydrolysis Probes targeting the *CXXC5* gene on a Light Cycler 480 machine (Roche) in accordance with the manufacturer's instructions of the kit Lightcycler® 480 ProbesMaster (Cat. N° 04 707 494 001). Relative mRNA expressions were normalized to rpP2 gene expression in a two-colour duplex reaction. Primers for detection of *CXXC5* (5'-tccgctgctctggagaag-3' (Seq. ID No. 4), 5'-cacacgagcagtgacattgc-3' (Seq. ID No. 5) and 6FAM-aacccaaagctgccctctcc-BBQ (Seq. ID No. 8)) and *rpP2* (5'-atgcgctacgtcgcc-3' (Seq. ID No. 6), 5'-ttaatcaaaaaggccaaatcccat-3' (Seq. ID No. 7) and Cy5-agctgaatggaaaaaacattgaagacgtc-BBQ (Seq. ID No. 9)), were designed to be used in the same conditions of real-time PCR amplification. After initial denaturation at 95°C for 5 min, samples were run through 44 cycles of the following conditions: denaturation for 10 seconds at 95 °C and elongation at 55 °C for 20 seconds.

### Analysis of CXXC5 mRNA expression in a microarray dataset and correlation analysis with the overall survival of acute myeloid leukaemia patients with normal cytogenetics.

One microarray dataset for which (1) *CXXC5* mRNA expression data, and (2) survival data were available online, was downloaded from the Gene Expression Omnibus website (http://www.ncbi.nlm.nih.gov/geo/) with accession number GSE12417. This microarray dataset was originally performed by Metzeler *et al.,* see above in order to develop a gene expression signature to predict treatment outcome for acute myeloid leukaemia patients with normal karytotype (n=163 patient samples). For each microarray platform (Affymetrix GeneChip^{®} Human Genome U133A, Affymetrix GeneChip^{®} Human Genome U133B, and Affymetrix GeneChip^{®} Human Genome U133 Plus 2.0), the whole raw gene expression data were normalized using RMA with the Expression Console software from Affymetrix. The *CXXC5* mRNA expression was calculated as the mean intensity of the 3 probe sets targeting *CXXC5* (222996_s_at, 224516_s_at and 233955_x_at). Gene expression data were also extracted for some already approved prognosis factors WT1 (206067_s_at and/or 206067_s_at), MN1(205330_at), BAALC (222780_s_at), ERG (241926_s_at), MECOM (EVI, 226420_at). Patients were then clustered into two (above or below median) or three (high, intermediary or low *gene* expression) groups according to their *CXXC5* mean intensity. The patient groups with high or low *CXXC5* mRNA (RINF) were then correlated with the clinical outcomes concerning survival that were also accessed through the Gene Expression Omnibus website ("E-GEOD-12417.sdrf.xls" file). The Kaplan-Meier curves (for survival analysis) and the log-rank test were performed by using the statistical SPSS 15.0.

### Detection of RINF by antibody

The expression of RINF can also be determined by an antibody approach. Western blotting was carried out as previously described by Wu YL, Dudognon C, Nguyen E, et al. Immunodetection of human telomerase reverse-transcriptase (hTERT) reappraised: nucleolin and telomerase cross paths. J Cell Sci. 2006;119:2797-2806.

Customized rabbit polyclonal peptide-specific antibody against RINF was produced by Biogenes GmBH. The immunogenic peptide corresponds to amino acids 45-58 of the RINF protein. Antibody specificity was confirmed by competitive inhibition of the western-blot signal by addition of the immunogene peptide to the primary antibody solution. Briefly, blots were incubated with primary antibody against RINF (polyclonal antibody), and then with an appropriate peroxydase conjugated secondary antibody. Detection of protein was performed using a chemiluminescent detection system (Amersham Pharmacia Biotech).

### Kit

For the expression studies of the above mentioned gene/protein, RINF, any method for quantitatively detecting the amplified product can be used, including, for example using any fluorescent dyes or labeled probes and standard methods.

Quantification of a sample containing an unknown number of target sequences typically is carried out with reference to a "standard curve" generated from a series of amplifications of samples containing the target sequence in a range of known amount. The standard curve is used to calculate an input copy number from the signal generated during an amplification. Thus, the unknown target sequence copy number in the sample of interest is estimated using the standard curve calculating the copy number that previously was determined to yield a signal equal to that observed. The concentration of the target sequence in the sample then is calculated from the input copy number and the sample size, which is determined prior to the reaction.

Quantitative estimates can be sensitive to variability in either the input sample size or in the reaction efficiency. The effect of inter-reaction variability of the input sample size on the calculated RINF concentration can be eliminated by using a control gene. A control gene is selected which provides an independent measure of the amount of RNA in the sample. The calculated concentration of the RINF mRNA is adjusted based on the independent measure of the sample.

Variability in the amplification efficiency between the reactions used to generate the standard curve and the reaction used to assay the sample of interest can affect the applicability of the standard curve. Carrying out the reactions used to generate the standard curve simultaneously with the reaction used to assay the sample of interest, using the same "master mix" of amplification reagents, and, preferably, in adjacent wells in the same thermal cycler, will minimize the inter - reaction variation in efficiency. Alternatively, an in thermal standard can be used to adjust the results to account for variation in amplification efficiency.

The effect of inter - reaction variability of reaction efficiency between the reactions used to generate the standard curve and the reaction used to assay the sample of interest can be eliminated by using a sample of interest and is amplified with internal standard. The internal standard is added to reaction in a known copy number and co-amplified along with the RINF mRNA target. The signal generated from the known amount of the internal standard provides an indication of the overall reaction efficiency which can be used to adjust the estimate difference in copy number to account for the difference in reaction efficiencies.

The internal standard is a nucleotide sequence that contains the same primer binding sites present in the target such that it is amplified by the same primer pair, but is distinguishable from the target sequence either by length or, preferably, by the presence of a unique internal sequence. The internal standard is included in a known copy number amplifications of the sample of interest and is amplified with approximately the same efficiency as the target sequence. Any change in the signal generated by amplification of the internal standard relative to the signal expected from the standard curve reflects a change in the overall reaction efficiency and is used to adjust the estimate of the target sequence copy number correspondingly.

The fourth aspect of the disclosure relates to kits comprising useful components for practicing the present method: A useful kit can contain oligonucleotide primers and, optionally, probes specific for the targets regions of the RINF mRNA described. Other optional components of the kit include, for example, any standard products known by skilled scientist in the field for the quantitative RT-PCR amplification like, agents to catalyze the synthesis of primer extension (enzymes, buffer), the deoxynucleotide triphosphates (dNTP), internal standard nucleic acids, a reference RNA (positive control), non - amplified control nucleic acid (negative control), appropriate buffers for PCR or hybridization reactions, and instructions for carrying out the present method. For examples, the reference control can be RNA from cell lines such as NB4, K562, HL60, or MCF7.

Another kit, or other component in the same kit, can contain SYBR green, oligonucleotide primers and, optionally, specific for the targets regions of the RINF. Other components of the kit include, substrate nucleotide triphosphates, internal standard, agents to catalyze the synthesis of primer extension, positive control, and negative control.

The examples of the present invention presented here are provided only for illustrative purposes and not to limit the scope of the invention.

### Results

The invention and the results of the experiments leading to the invention will now be further described, with reference to the following figures:

Figure 1 shows that high *RINF* mRNA expression is related to bad prognosis in AML with normal karyotype CN-AML (n=163). In these cohorts, the cumulative survivals (Kaplan-Meier) are represented according to the *RINF* mRNA expression levels detect by affymetrix arrays (HG-U133B or U133 plus 2.0). The patients have been classified in two equivalent groups (A), according to an expression above (High, n=81) or below (Low, n=81) the median, or in 3 equivalent groups (B and C), according to a High (n=55), an Intermediary (n=54), or a Low (n=54) *RINF* expression level. Note that because of an odd number of patients (n=163), the patient at the median has been censored for the 2 groups stratification and were not exactly equivalents for the 3 groups stratification (n=55 *versus* 54). *P values* (log-rank test) of the comparison of the various groups of patients are indicated in the panels.

Figure 2 to 5 summarizes the results of the GSE23312 study on AML. In this study, data for individuals suffering from AML with all cytogenetic subgroups are available. As shown in figures 2, 3, 4, and 5, low RINF mRNA expression is related to a better prognosis in all subtypes of AML, including normal karyotype, as well as with inversion or translocation (AML-CBF).

Figure 6a and 6b show the prediction of survival outcomes by gene expression measurement of prognosis factors WT1, MN1, BAALC, ERG, MECOM (EVI1) in the same cohort of AML patients with normal karyotype CN-AML (n=163). Gene mRNA expressions were extracted with the specific probesets WT1 (206067_s_at and/or 206067_s_at), MN1(205330_at), BAALC (222780_s_at), ERG (241926_s_at) and MECOM (EVI, 226420_at). Cumulative survivals (Kaplan-Meier) are represented according to mRNA expression levels detect by affymetrix arrays (HG-U133A, HG-U133B or U133 plus 2.0). Like for RINF mRNA, the patients have been classified in two equivalent groups (A), according to an expression above (High, n=81) or below (Low, n=81) the median, or in 3 equivalent groups (B and C), according to a High (n=55), an Intermediary (n=54), or a Low (n=54) RINF expression level. Note that because of an odd number of patients (n=163), the patient at the median has been censored for the 2 groups stratification and were not exactly equivalents for the 3 groups stratification (n=55 versus 54). P values (log-rank test) of the comparison of the various groups of patients are indicated in the panels. Notably, as shown on figure 2a and 2b, none of these already approved prognosis factors was as efficient as RINF to predict the survival outcome (as indicated by the P values) of these AML patients with normal karyotype.

Moreover, a cohort of ALL patients was tested wether the RINF mRNA expression could correlate with the risk of relapse. For all the ALL patients (n=197) of this cohort, RINF expression data were analyzed by microarray analysis (study GSE13576) as described for the AML cohort (GSE12417). As shown in Figure 7, the median RINF mRNA expression was not equivalent in the different group of patients that relapsed or did not relapsed. Moreover, as shown in table 1 high RINF mRNA expression is associated with a higher risk of relapse (11,7 % vs. 4,6 %), and more especially of early relapse (9,1 % vs. 3 %). A high RINF mRNA expression predicts a three fold increased risk of early relapse compared to low RINF expression. High and low RINF mRNA expression, respectively, represents the group of patients with a RINF mRNA expression above or below the median.

**table 1 acute lymphocytic leukaemia (ALL), GSE13576**

| | **High RINF (n=98)** | **Low RINF (n=99)** | **Total (n=197)** |
|---|---|---|---|
| No relapse (%) | 75 (38 %) | 90 (45,7 %) | 165 |
| Relapse (%) | 23 (11,7 %) | 9 (4,6 %) | 32 |
| - Early relapse | 18 (9,1 %) | 6 (3 %) | 24 |
| - Late relapse | 5 (2,6 %) | 3 (1,5 %) | 8 |

From the above results it is clear that RINF represents a measure allowing for determining the progression of the disease or a prognosis for survival of the patient with leukaemia, in particular, acute and chronic lymphocytic/lymphoblastic or myeloid leukaemia, or the risk of a relapse or leukaemia in a patient diagnosed and, optionally, treated for leukaemia. In addition, RINF allows to stratify therapy regimens of patients afflicted with leukaemia. This is in contrast to other markers described so far. For example, as shown herein, other markers described in the art, like MN1 or WT1 do not allow to identify the different groups of patients afflicted with leukaemia, or at least in a lower extend that it is possible for RINF and, thus, do not allow to judge on a higher risk of relapse or on the progression of the disease as well as on the prognosis for survival and for stratifying the therapy regimens as it is possible based on RINF. In Figure 7 RINF mRNA expression (at a diagnosis time) is higher in the group of acute lymphocytic leukaemia patients (n=197) that relapse after treatment than in persons showing no relapse.

Furthermore, studies on chronic lymphoid leukaemia (CLL) (microarray study GSE10138) demonstrated that high RINF mRNA expression is associated with a higher risk of progression, see table 2, below:

**Table 2**

| | **High RINF (n=6)** | **Low RINF (n=62)** | **Total (n=68)** |
|---|---|---|---|
| Stable (%) | 1 (16.7%) | 35 (57.1%) | 36 (53%) |
| Progressive (%) | 5 (83.3%) | 27 (42.9%) | 32 (47%) |

Moreover, in chronic myeloid leukaemia based on the data of GSE4170, RINF expression is increased during the progression of CML from chronic phase to blast crisis, see figure 8. The RINF (CXXC5) gene expression data were extracted from a gene list (supporting table 4 or 10423Table4.xls) available online at the PNAS website (http://www.pnas.org/content/103/8/2794/suppl/DC1). CP: chronic phase (n=42), AP: accelerated phase by blast count criteria (n=9) or by the occurrence of additional clonal cytogenetic changes (n=8), BC: blast crisis (n=28). Thus, RINF expression may be used as marker for identifying progression steps in chronic myeloid but also lymphocytic leukaemia.

Quantifying the relative mRNA level of RINF in neoplastic cells from patients with a leukaemia can help doctors determine whether or not a tumour is of good or bad prognosis, as well as if a certain treatment is likely to succeed. In the case of leukaemia, and especially in the case of AML, these prognostic indicators are important for the physicians to distinguish the group of patients that would more likely benefit from a chemotherapy (usually, disease with a favourable prognosis) from patients that would not and should be considered for stem cell transplantation (usually, patients with unfavourable prognosis and age below 60).

Our results demonstrate that quantification of RINF mRNA expression could be a significant predictor of patient survival and leukaemia progression.

Indeed, by extracting the *CXXC5* mRNA expression data from several independent microarray study available in public databases, and correlating it with the clinical outcome, we have tested and demonstrated for the first time, a strong correlation, between a high *RINF* mRNA expression and low survival rate for patients suffering from leukaemia, more precisely AML, ALL, CLL, and CML, more precisely AML with normal cytogenetics as well as with AML- CBF (translocation t(8;21) or inv(16)).

At the best of our knowledge, no previous study has examined the relationship between the RINF mRNA expression and a patient's prognosis in leukaemia. This inventive idea was tested because we have been the first to observe that this gene was playing a important role into differentiation of myeloid cells and was deregulated in cancer cell types. However, and importantly, these results were not obvious because many genes, shown to be de-regulated in cancer, have in fact no predictive value toward a patient survival or response to therapy. Furthermore, while WO2009/151337describe that the induction of RINF mRNA expression (by retinoic acid) correlated with a growth arrest of the leukemic cell model NB4 in vitro of Acute Promyelocytic Leukemia (AML-with t(15;17)) (and the clinical remission relapse of the patients). Here, we surprisingly found the opposite trend in leukemia (AML, ALL, CLL), since the high RINF expression at diagnosis correlates with a more agressive phenotype of the leukemic cells (a shorter survival rate of patients, or a lower probability of response to therapy, or a higher probability to relapse, or a higher probability to progress into disease at higher risk).

Moreover, it was not "obvious" also because we did not find this trend in an other malignant haemopathies (myeloma) in which High RINF does not seem to correlate with the survival. This is an important area of research in cancer because there are little data to help physicians chose which treatment is best for which patients or to determine when a change in treatment is warranted.

The RINF mRNA level can be measured in the biological sample including blood, liquor, bone marrow, tumour, or tumour biopsy, taken out at the time of surgery by using the kit and methods according to the present disclosure.

Potentially, this tool could be useful in the clinic for clinicians to take their decisions about whether a treatment is helping. Our ultimate goal is to continue effective treatment, but spare patients the side effects of a treatment that is destined to fail.

To conclude, the present disclosure provides a marker with superior properties over prior art marker allowing to determine the progression of leukaemia in a patient afflicted therewith or allow to provide a prognosis for survival of a patient with leukaemia. In addition, the present disclosure allows to determine the risk of a relapse of leukaemia in a patient diagnosed and, optionally, treated for leukaemia based on RINF expression. Further, the present disclosure allows to stratify therapy regimens of individuals afflicted with leukaemia. Moreover, as demonstrated herein in all four major types of leukaemia, ALL, CLL, AML, CML, RINF represents a suitable marker, and, strikingly, by comparing RINF for the first time in the same cohort with other well known markers in the art, like MN1, WT1, ERG, MECOM or BAALC, RINF has a clearly superior capability to predict the survival over these other markers.

## Claims

1. A method for determining the progression of the disease or a prognosis for survival of a patient with acute myeloid leukaemia, or the risk of a relapse of acute myeloid leukaemia in a patient diagnosed and, optionally, treated for acute myeloid leukaemia, **characterized in that** said method comprising:
a) determining the level of CXXC5 mRNA expression in a biological sample. from said patient,
b) comparing the level of CXXC5 mRNA expression in said sample to a reference level of CXXC5,
whereby a level of CXXC5 mRNA expression in said sample of said patient lower than the reference level correlates with better prognosis and increased survival of said patient, slower progression of the disease or lower risk of a relapse, and whereby a level of CXXC5 mRNA expression in said sample higher than the reference level correlates with decreased survival and bad prognosis, faster progression of the disease or higher risk of relapse.

2. The method according to claim 1, wherein the acute myeloid leukaemia is acute myeloid leukaemia with normal karyotype.

3. The method according to any one of the preceding claims wherein said biological sample is provided from bone marrow, blood or tissue, like plasma, serum, bone marrow, tumour, tumour biopsy or neoplastic cell containing sample.

4. The method according to any one of the preceding claims wherein the level of CXXC5 is determined by quantification of the expression of mRNA encoding for CXXC5 or a quantification of a nucleic acid comprising a sequence of SEQ ID No. 1 or SEQ ID No. 2.

5. The method according to claim 4 wherein the level of CXXC5 is determined by quantitative RT-PCR whereby at least parts of SEQ ID No. 1 or SEQ ID No. 2 are amplified, optionally wherein said RT-PCR uses primers amplifying exon 3 and 4, or exon 1 and 2 of SEQ ID Nos. 1 to 2, and/or wherein the level of CXXC5 expression is determined based on the relative expression of CXXC5 normalized to the expression of the gene rpP2 also known as RPLP2 (acidic ribosomal phosphor protein P2).

6. The method according to claim 4 wherein the primers and probe for determining the level of SEQ ID No. 1 or 2 are:
5'-tccgctgctctggagaag-3' (Seq. ID No. 4), 5'-cacacgagcagtgacattgc-3' (Seq. ID No. 5) and 6-FAM-aacccaaagctgccctctcc-BBQ (Seq. ID No. 8), or
the primers and probe for determing the level of rpP2 are:
5'-atgcgctacgtcgcc-3' (Seq. ID No. 6), 5'-ttaatcaaaaaggccaaatcccat-3' (Seq. ID No. 7) and Cy5-agctgaatggaaaaaacattgaagacgtc-BBQ (Seq. ID No. 9).

7. A method according to any one of the preceding claims wherein said method further comprises the step
c1) classifying said patients as belonging to either a first or a second group of patients, wherein said first group of patients having levels of CXXC5 mRNA expression lower than the reference level is classified as having an increased likelihood of survival, slower progression of the disease, better prognosis or lower risk or relapse compared to said second group of patients having levels of CXXC5 mRNA expression higher than the reference level; or
c2) classifying said patient as belonging to either a first or a second group of patients, wherein said first group of patients having levels of CXXC5 mRNA expression lower than said second group of patients, and where the first group of patients is classified as having an increased likelihood of survival, slower progression of the disease, better prognosis or lower risk or relapse compared to said second group of patients.

8. The method according to claim 1 wherein when comparing the level of CXXC5 mRNA expression prior to the treatment with the level of CXXC5 mRNA expression after or during treatment indicates the effectiveness of said treatment wherein a decrease in the level of CXXC5 mRNA expression after treatment indicates the treatment is effective against said acute myeloid leukaemia.

9. The method according to any one of the preceding claims wherein the control sample or reference sample is from an individual not afflicted with acute myeloid leukaemia.

## Patentansprüche

1. Verfahren zur Bestimmung der Progression der Erkrankung oder einer Prognose für das Überleben eines Patienten mit akuter myeloischer Leukämie oder dem Risiko eines Rezidivs der akuten myeloischen Leukämie in einem Patienten der darauf diagnostiziert und gegebenenfalls behandelt wurde, **dadurch gekennzeichnet, dass** das Verfahren umfasst
a) Bestimmen des Niveaus der CXXC5 mRNA Expression in einer biologischen Probe dieses Patienten,
b) Vergleich des Niveaus der CXXC5 mRNA Expression in dieser Probe mit einem Referenzniveau von CXXC5,
wobei wenn das Niveau der CXXC5 mRNA Expression in dieser Probe dieses Patienten niedriger als das Referenzniveau mit einer besseren Prognose und einer erhöhten Überlebensrate dieses Patienten, einem langsamen Fortschreiten der Erkrankung oder einem geringen Risiko eines Rezidivs korreliert, und wobei ein höheres Niveau an CXXC5 mRNA Expression in dieser Probe als das Referenzniveau mit einer verschlechterten Überlebensrate und einer schlechteren Prognose, einer schnelleren Progression der Erkrankung oder einem höheren Risiko eines Rezidivs korreliert.

2. Verfahren nach Anspruch 1, wobei die akute myeloische Leukämie eine akute myeloische Leukämie mit normalem Karyotyp ist.

3. Verfahren nach einem der vorherigen Ansprüche, wobei die biologische Probe bereitgestellt wird aus Knochenmark, Blut oder Gewebe, wie Plasma, Serum, Knochenmark, Tumor, Tumorbiopsie, oder einer neoplastische Zellen enthaltenen Probe.

4. Verfahren nach einem der vorherigen Ansprüche, wobei das CXXC5 Niveau bestimmt wird durch Quantifizieren der Expression der mRNA kodierend für CXXC5 oder der Quantifizierung einer Nukleinsäure umfassend eine Sequenz von SEQ ID Nr. 1 oder SEQ ID Nr. 2.

5. Verfahren nach Anspruch 4, wobei das CXXC5 Niveau bestimmt wird mittels quantitativer RT-PCR wobei mindestens Teile der SEQ ID Nr. 1 oder SEQ ID Nr. 2 vervielfältigt werden, gegebenenfalls wobei diese RT-PCR Primer verwendet die Exon 3 und Exon 4, oder Exon 1 und Exon 2 der SEQ ID Nr. 1 bis 2 vervielfältigen und/oder wobei das Niveau der CXXC5 Expression bestimmt wird auf Basis der relativen Expression von CXXC5 normalisiert zu der Expression des Gens rpP2 auch bekannt als RPLP2 (saures ribosomales Phosphorprotein P2).

6. Verfahren nach Anspruch 4, wobei die Primer und Probe zur Bestimmung des Niveaus an SEQ ID Nr. 1 oder 2 sind:
5'-tccgctgctctggagaag-3' (SEQ ID Nr. 4), 5'-cacacgagcagtgacattgc-3' (SEQ ID Nr. 5) und 6-FAM-aacccaaagctgccctctcc-BBQ (SEQ ID Nr. 8), oder die Primer und Proben zur Bestimmung des Niveaus an rpP2 sind:
5'-atgcgctacgtcgcc-3' (SEQ ID Nr. 6), 5'-ttaatcaaaaaggccaaatcccat-3' (SEQ ID Nr. 7) und Cy5-agctgaatggaaaaaacattgaagacgtc-BBQ (SEQ ID Nr. 9).

7. Verfahren nach einem der vorherigen Ansprüche, wobei das Verfahren weiterhin den Schritt umfasst
c1) Klassifizieren dieser Patienten dass sie zu einer ersten oder zweiten Patientengruppe gehören, wobei diese erste Patientengruppe als solche mit CXXC5 mRNA Expressionsniveaus niedriger als das Referenzniveau klassifiziert werden, die eine erhöhte Wahrscheinlichkeit des Überlebens, eines geringeren Fortschreitens der Erkrankung, einer besseren Prognose oder eines geringeren Rezidivrisikos aufzeigen im Vergleich zu der zweiten Gruppe von Patienten deren Niveau an CXXC5 mRNA Expression höher ist als das Referenzniveau; oder
c2) Klassifizieren des Patienten dass sie zu einer ersten oder zweiten Patientengruppe gehören, wobei diese erste Patientengruppe Niveaus an CXXC5 mRNA Expression aufweisen kleiner als die der zweiten Patientengruppe wobei die erste Patientengruppe klassifiziert wird als eine mit einer verbesserten Wahrscheinlichkeit des Überlebens, einer geringeren Progression der Erkrankung, einer besseren Prognose oder geringerem Risiko eines Rezidivs im Vergleich zu der zweiten Gruppe der Patienten.

8. Verfahren nach Anspruch 1, wobei wenn die Niveaus der CXXC5 mRNA Expression vor der Behandlung mit den Niveaus an CXXC5 mRNA Expression nach oder während der Behandlung verglichen werden die Wirksamkeit der Behandlung anzeigt wird, wobei eine Erniedrigung des Niveaus an CXXC5 mRNA Expression nach Behandlung anzeigt, dass die Behandlung wirksam war gegenüber der akuten myeloischen Leukämie.

9. Verfahren nach einem der vorherigen Ansprüche, wobei die Kontrollprobe oder Referenzprobe von einem Individuum stammt, das nicht von akuter myeloischer Leukämie betroffen ist.

## Revendications

1. Procédé de détermination de la progression de la maladie ou d'un pronostic de survie d'un patient atteint de leucémie myéloïde aiguë, ou du risque d'une récidive de leucémie myéloïde aiguë chez un patient diagnostiqué et, facultativement, traité pour la leucémie myéloïde aiguë, **caractérisé en ce que** ledit procédé comprend .
a) la détermination du taux d'expression d'ARNm CXXC5 dans un échantillon biologique provenant dudit patient,
b) la comparaison du taux d'expression d'ARNm CXXC5 dans ledit échantillon à un taux de référence de CXXC5, de sorte qu'un taux d'expression d'ARNm CXXC5 dans ledit échantillon dudit patient inférieur au taux de référence soit corrélé à un meilleur pronostic et augmente la survie dudit patient, une progression plus lente de la maladie ou un risque plus faible de récidive, et de sorte qu'un taux d'expression d'ARNm CXXC5 dans ledit échantillon plus élevé que le taux de référence soit corrélé à une survie réduite et un pronostic médiocre, une progression plus rapide de la maladie ou un risque plus élevé de récidive.

2. Procédé selon la revendication 1, dans lequel la leucémie myéloïde aiguë est une leucémie myéloïde aiguë avec un caryotype normal.

3. Procédé selon l'une quelconque des revendications précédentes dans lequel ledit échantillon biologique est obtenu à partir de moelle osseuse, de sang ou de tissu, tel que le plasma, le sérum, la moelle osseuse, une tumeur, une biopsie de tumeur ou un échantillon contenant des cellules néoplasiques.

4. Procédé selon l'une quelconque des revendications précédentes dans lequel le taux de CXXC5 est déterminé par une quantification de l'expression d'ARNm codant pour CXXC5 ou une quantification d'un acide nucléique comprenant une séquence de SEQ ID No. 1 ou SEQ ID No. 2.

5. Procédé selon la revendication 4 dans lequel le taux de CXXC5 est déterminé par RT-PCR quantitative de sorte qu'au moins des parties de SEQ ID No. 1 ou SEQ ID No. 2 soient amplifiées, facultativement dans lequel ladite RT-PCR utilise des amorces amplifiant les exons 3 et 4, ou les exons 1 et 2 de SEQ ID No. 1 à 2,
et/ou dans lequel le taux d'expression de CXXC5 est déterminé sur la base de l'expression relative de CXXC5 normalisée par rapport à l'expression du gène rpP2 également appelé RPLP2 (phosphoprotéine ribosomique acide P2).

6. Procédé selon la revendication 4 dans lequel les amorces et la sonde pour déterminer le taux de SEQ ID No. 1 ou 2 sont :
5'-tccgctgctctggagaag-3' (Seq. ID No. 4), 5'-cacacgagcagtgacattgc-3' (Seq. ID No. 5) et 6-FAM-aacccaaagctgccctctcc-BBQ (Seq. ID No. 8), ou
les amorces et la sonde pour déterminer le taux de rpP2 sons :
5'-atgcgctacgtcgcc-3' (Seq. ID No. 6), 5'-ttaatcaaaaaggccaaatcccat-3' (Seq. ID No. 7) et Cy5-agctgaatggaaaaaacattgaagacgtc-BBQ (Seq. ID No. 9).

7. Procédé selon l'une quelconque des revendications précédentes, ledit procédé comprenant en outre l'étape de
c1) classification desdits patients comme appartenant à un premier ou un deuxième groupe de patients, ledit premier groupe de patients ayant des taux d'expression d'ARNm CXXC5 inférieurs au taux de référence étant classé comme ayant une probabilité de survie augmentée, une progression plus lente de la maladie, un meilleur pronostic ou un risque de récidive plus faible par rapport audit deuxième groupe de patients ayant des taux d'expression d'ARNm CXXC5 plus élevés que le taux de référence ; ou
c2) classification dudit patient comme appartenant à un premier ou un deuxième groupe de patients, ledit premier groupe de patients ayant des taux d'expression d'ARNm CXXC5 plus faibles que ledit deuxième groupe de patients, et où le premier groupe de patients est classé comme ayant une probabilité de survie augmentée, une progression plus lente de la maladie, un meilleur pronostic ou un risque de récidive plus faible par rapport audit deuxième groupe de patients.

8. Procédé selon la revendication 1 dans lequel, lors de la comparaison du taux d'expression d'ARNm CXXC5 avant le traitement avec le taux d'expression d'ARNm CXXC5 après ou pendant le traitement indique l'efficacité dudit traitement dans lequel une diminution du taux d'expression d'ARNm CXXC5 après le traitement indique que le traitement est efficace contre ladite leucémie myéloïde aiguë.

9. Procédé selon l'une quelconque des revendications précédentes dans lequel l'échantillon témoin ou l'échantillon de référence est d'un individu non atteint de leucémie myéloïde aiguë.
